# EUROPEAN PATENT APPLICATION

(11) **EP 0 765 933 A2**
(43) Date of publication of application: **02.04.1997**
(21) Application number: 96114689.1
(22) Date of filing: 13.09.1996
(51) Int. Cl.: C12M 1/24, C12M 3/06, C12M 3/04

(54) **Growth environment assembly and method of use thereof**

(30) Priority: 29.09.1995 US 536339
(71) Applicant: Becton Dickinson and Company, Franklin Lakes, New Jersey 07417-1880 (US)
(72) Inventor: O'Leary, Robert K., Lexington, M.A. 02173 (US); LaRocca, Paul J., Pepperell, M.A. 01463 (US); Stevens, Timothy A., Warwick, N.Y. 10990 (US)
(74) Representative: Selting, Günther, Dipl.-Ing.

(57) **Abstract**

A method and assembly for treating biological environments with varying doses of fluids *in vitro*. The assembly comprises a vessel, a closure and a tubular membrane suspended in the vessel, so as to provide rapid and uniform equilibration of fluids into the vessel. The assembly provides means for carrying out pharmacokinetic and toxicokinetic studies.

## Description

### BACKGROUND OF THE INVENTION

### 1. Field of the Invention

This invention relates to a method and assembly for treating living and non-living biological environments with varying doses of fluids, solutes, drug molecules or diffusible agents *in vitro*, and more particularly to a vessel and closure assembly having means for releasing and/or delivering fluids, solutes, drug molecules or diffusible agents in environments that may include cell lines and tissue cultures.

### 2. Description of Related Art

The use of *in vitro* cell lines and tissue cultures in carrying out pharmacokinetic and toxicokinetic studies related to safety evaluations of new drugs is a growing area of scientific development. By using cell lines and tissue cultures, the need for large animal studies is reduced.

Pharmacokinetic and toxicokinetic studies depend upon the control rate of drug delivery and dose of drug to a cell line for multiple drugs simultaneously. Repeated drug administration can influence the toxicity of the drug by changing its metabolism and stimulating the cell's synthesis of certain proteins that can effect the drug's activity on the cell line.

Current technologies for studying *in vitro* pharmacokinetics and toxicokinetics use either a closed monolayer culture system (MCS) or a dynamic perfused cell system (DPCS). The MCS employs an immediate total dose application of the test drug. Its limitation is that it operates in essentially a single dose range which can be toxic due to over dose.

On the other hand, the DPCS acts as an *in vivo* circulating model. The *in vitro* routes of bolus administration does not mimic the *in vivo* method of drug administration since their are no tissue masses to act as the sustain release matrix.

A need exists for a delivery system in order to carry out *in vitro* pharmacokinetic metabolic pathway studies of drugs, proteins, growth factors and other such biologicals. The need arises because it is experimentally undesirable to deliver fluids, such as drugs to cell lines and tissue cultures in bolus concentrations. Bolus concentrations cause the pharmacological dose to quickly and uncontrollably extend into the toxicological range.

Therefore, a special need exists for a drug delivery and/or releasing system which permits the control of drug delivery and/or release and provides an assessment of drug concentration on cell lines and tissue culture environments so as to establish a correlation of drug action with adverse reactions.

Such a need for a new type of drug delivery and/or releasing system for treating biological environments has not been suggested or taught in the literature.

### SUMMARY OF THE INVENTION

The present invention is a method and assembly for delivering and/or releasing fluids, solutes, drug molecules or diffusible agents to environments, such as living and non-living biological environments that may include such as cell lines and tissue cultures.

Most preferably, the present invention is an assembly comprising a vessel, a closure and means for delivering fluids, such as drugs, proteins, growth factors and other such biologicals into the vessel. Most preferably, the means for delivering fluids into the vessel is at least one tubular membrane extending through and suspended into the vessel.

Preferably, the vessel is a flask, roller bottle, tube, spinner flask, stirred bioreactor or any vessel that will facilitate cell culture viability. Most preferably, the vessel is a flask or roller bottle. Vessels that are applicable to this invention include those described in U.S. Patent Nos. 5,272,084; 4,770,854; 5,139,952; 4,334,028; 4,289,248; 4,387,822; and 5,047,347, which are incorporated by reference.

Desirably, the closure is a cap, push cap, threaded cap, screw cap or stopper.

Preferably, the tubular membrane extends from an entry port located on the vessel and ends at an evacuation port preferably located on the vessel opposite the entry port. The tubular membrane comprises a first end and a second end, wherein the first end is connected to the entry port and the second end is connected to the evacuation port. The tubular membrane comprises a hollow cavity containing the means to distribute fluid substances.

The evacuation port provides the means for the drainage and/or withdrawal of excess fluid that is not distributed by the membrane into the vessel. Therefore, stagnation of fluid in the membrane is minimized and efficiency of fluid flow through the membrane is enhanced.

Most notably, the assembly of the present invention provides a continuous controlled release of fluids which diffuse from the membrane and into the vessel with minimal stagnation of fluid in the membrane. The present invention eliminates the delivery of bolus concentrations of fluids which causes the pharmacological dose to quickly and uncontrollably extend into the toxicological range.

The tubular membrane is an imperfect barrier separating two fluids and hinders free diffusion of a substance in an isotropic medium. Diffusion is the tendency of molecules to migrate from a region of high concentration (potential and/or activity) to a region of lower concentration (potential and/or activity).

The use of the assembly of the present invention permits the control of fluid delivery and concentration to cell lines and tissue cultures and permits the development of steady state drug diffusion for studying possible mechanisms for the passage of nutrients and drugs and for studying *in vitro* pharmacokinetic studies on living cells.

The present invention provides important advances over methods used to perform pharmacokinetic and toxicokinetic studies. The assembly of the present invention provides the ability to maintain an *in vitro* culture system wherein the cells are subjected to an environment which permits them to function in a manner closely simulating that encountered *in vivo*. The invention also permits the efficient removal of desired products from the cells being cultured and provides important advantages in the efficiency, economy and flexibility of operation.

An alternate embodiment of the assembly of present invention comprises multiple tubular membranes extending through the vessel so that more than one drug solute may be injected into the tubular membrane at one time or at varying time intervals.

A further advantage of the present invention include its use in drug therapy by providing quantitative evaluations of cell populations responding to drug therapy. Whereby, kinetic rate constants, activation energies, entropies and other basic pharmacological parameters can be evaluated. Another feature of the present invention is that metabolic substances such as uremic poisons can be delivered to cells for the purpose of investigating the effects of metabolites on *in vitro* cultured cells.

### DESCRIPTION OF THE DRAWINGS

FIG. 1 is a perspective view of the assembly of the present invention.

FIG. 2 is an isolated cross sectional view of the tubular membrane shown in FIG. 1.

FIG. 3 is a perspective view of an alternate embodiment of the present invention.

### DETAILED DESCRIPTION

While this invention is satisfied by embodiments in many different forms, there is shown in the drawings and will herein be described in detail, the preferred embodiments of the invention, with the understanding that the present disclosure is to be considered as exemplary of the principles of the invention and is not intended to limit the invention to the embodiments illustrated. Various other modifications will be apparent to and readily made by those skilled in the art without departing from the scope and spirit of the invention. The scope of the invention will be measured by the appended claims and their equivalents.

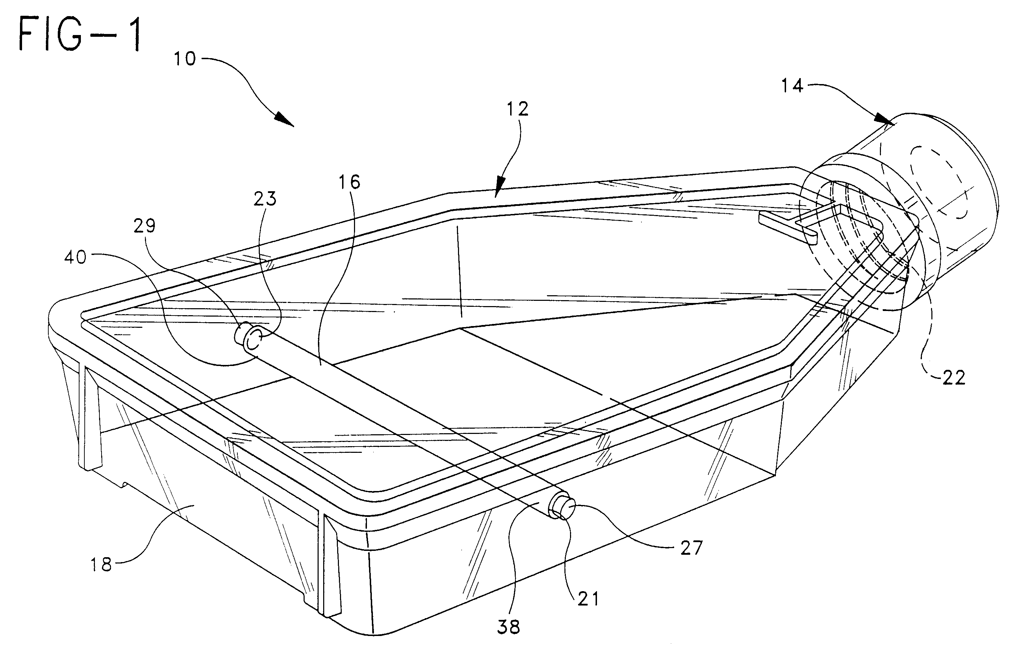

FIG. 1 illustrates an assembly **10**, comprising a vessel **12**, a closure **14** and tubular membrane **16**. The vessel is preferably made from impact resistant plastic or glass which is gas impermeable, optically clear, non-toxic and inert with respect to the cells to be cultured.

Vessel **12** has a body **18** in which material is adapted to be held until such time as the same is withdrawn or dispensed. It is unimportant whether body **18** is made of a collapsible or no-collapsible material, such as metals, plastics or glass.

As shown in FIG. 1, vessel **12** includes a neck **22** to receive closure **14**. Neck **22** is integral with the vessel and defines a cylindrical conduit having one end integral with the vessel and the other end defining an opening through which the cells and culture fluids may be introduced into the body of the vessel. Neck **22** and closure **12** constitute one of a number of well known means for introducing materials such as mammalian cells and culture fluids into body **18**. As is conventionally know, closure **14** is removed from neck **22** to provide an opening through which cells and culturing fluids can be introduced into the vessel. The closure is subsequently remounted onto the neck to re-seal the vessel.

The vessel further includes an evacuation port **21** and an entry port **23** that each preferably include a pierceable self sealing material. The tubular membrane extends from entry port **23** into the vessel and ends at evacuation port **21**. Additionally, a plug **27** and **29** may be located in the evacuation port and entry port on the outer surface of the vessel to maintain the conditions of the environment in the vessel.

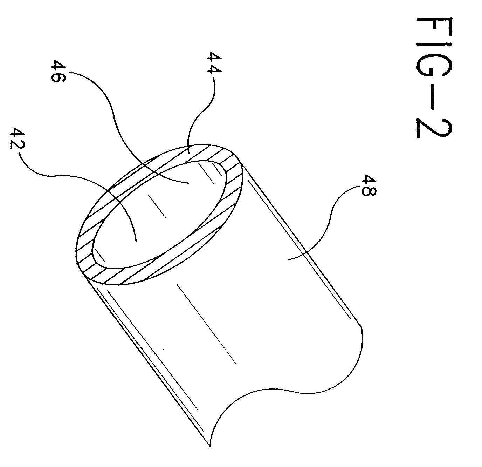

As shown in FIGS. 1 and 2, tubular membrane **16** comprises a first end **38**, a second end **40** and a hollow cavity **42** containing the means to distribute fluid substances. Tubular membrane **16** comprises a sidewall **44** that extends from first end **38** to second end **40** and comprises an inner surface **46** and an outer surface **48**.

Most preferably, the entry port and evacuation port are made of a pierceable self sealing material whereby entry can be made into the tubular membrane through entry port **23** by means for example by a hypodermic needle so that fluids can be injected into the tubular membrane and/or added or removed from the vessel without breaking the environment of the vessel. After the needle is removed, the self sealing material immediately reseals. The material is most preferably an elastomeric material.

Tubular membrane **16** may be made from any suitable gas permeable material so long as it provides for the passage of fluids such as drugs and biological fluids into vessel **12**. Preferably, the tubular membrane is made from a natural or synthetic polymer or a dialysis material.

Diffusion is characterized by the tendency of molecules to migrate from a region of high concentration to a region of lower concentration, but is more rigorously stated with respect to chemical potentials or activities rather than concentrations. The membrane is an imperfect barrier separating two fluids and hinders free diffusion of a substance in an isotropic medium.

The solute permeability of the tubular membrane may be small, medium or large. An example of the solute permeability are as follows: (i) a small solute permeability is about 50 to about 5000 molecular weight (i.e., urea, or insulin); (ii) a medium size solute permeability is about 6000 to about 37,000 molecular weight (i.e., insulin, or heparin); and (iii) a large molecule permeability is about 150,000 antibodies.

Most preferably the tubular membrane is made of polypropylene. An example of a commercially available tubular membrane is Accurel PP capillary membranes (trademark of AKZO, Germany).

The present invention may be used to inject materials into the vessel by the tubular membrane at the entry port by means of a syringe or the like. Excess fluid is easily drained and/or withdrawn from the tubular membrane at the evacuation port. If desirable, further substances can be injected and/or withdrawn from the vessel through the tubular membrane.

The injectable feature of the tubular membrane permits the investigator to alter the kinetics of the tubular membrane diffusivity by pre-injecting the tubular membrane with surfactants or other solvents such as dimethyl sulfoxide to accelerate or control the drug release or diffusion process.

The tubular membrane may be used to distribute substances such as drugs, hormones, insecticides, feromones and repellents into the vessel. The tubular membrane provides a means for the controlled release of the active substances wherein the substrate consists of an asymmetric wall formed from polymers.

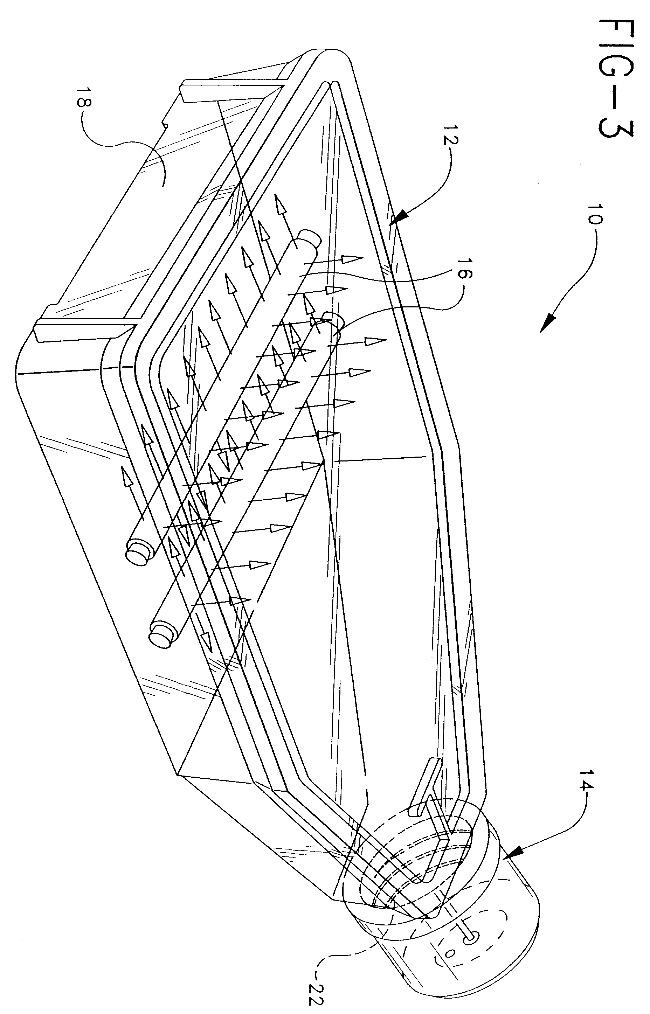

An alternate embodiment of the present invention as shown in FIG. 3, includes many components which are substantially identical to the components of FIG. 1. Accordingly, similar components performing similar functions will be numbered identically to those components of FIG. 1, except that a suffix "a" will be used to identify those similar components in FIG. 3.

Assembly **10a** as shown in FIG. 3 is an alternate embodiment of the present invention. The alternate embodiment of the invention as shown in FIG. 3, is that a first end **54** of a second tubular membrane **52** is attached to a second entry port **58** and the second end **62** is attached to a second evacuation port **64**. Each port may further include a plug such as **23a**.

Although FIG. 3 only shows two tubular membranes, it is well within the purview of the invention to have more than two tubular membranes extending through the vessel.

## Claims

1. An assembly for treating biological environments comprising:
a vessel comprising a chamber and a neck connected to said chamber having an opening for introducing cells and culture fluids into said chamber, an entry port located on said chamber and an evacuation port located on said chamber;
a closure for covering said opening in said neck comprising means for removably mounting said closure to said neck; and
means for delivering fluids into said vessel extending between said entry port and said evacuation port.

2. The assembly of Claim 1 wherein said means for delivering fluids into said vessel is at least one tubular membrane.

3. The assembly of Claim 2 wherein said tubular membrane comprises a first end connected to said entry port and a second end connected to said evacuation port.

4. The assembly of Claim 1 wherein said vessel is a flask or a roller bottle.

5. The assembly of Claim 1 wherein said closure is a cap or a stopper.

6. The assembly of Claim 1 wherein said entry port and said evacuation port comprise a pierceable self sealing material.

7. The assembly of Claim 3 wherein said tubular membrane is made of polypropylene.

8. The assembly of Claim 1 wherein said means for delivering fluids into said vessel is at least two tubular membranes.

9. The assembly of Claim 1 wherein said means for delivering fluids into said vessel is at least three tubular membranes.
